# EUROPÄISCHE PATENTSCHRIFT

(11) **EP 1 135 108 B1**
(45) Veröffentlichungstag und Bekanntmachung des Hinweises auf die Patenterteilung: **17.03.2004**
(21) Anmeldenummer: 99962207.9
(22) Anmeldetag: 03.12.1999
(51) Int. Cl.: A61K 9/16, A61K 9/50, A61K 9/20, B01J 2/00

(54) **VERFAHREN ZUR HERSTELLUNG VON AGGLOMERATEN MIT KERN-SCHALE-STRUKTUR**
METHOD FOR PRODUCING AGGLOMERATES COMPRISING A CORE-SHELL STRUCTURE
PROCEDE DE PRODUCTION D'AGGLOMERATS PRESENTANT UNE STRUCTURE DU TYPE NOYAU-COQUILLE

(30) Priorität: 04.12.1998 DE 19856149
(43) Veröffentlichungstag der Anmeldung: 26.09.2001
(73) Patentinhaber: BASF AKTIENGESELLSCHAFT, 67056 Ludwigshafen (DE)
(72) Erfinder: BERTLEFF, Werner, D-68519 Viernheim (DE); BRÖCKEL, Ulrich, D-67251 Freinsheim (DE); FRICKE, Helmut, D-67112 Mutterstadt (DE); HARZ, Hans-Peter, D-67373 Dudenhofen (DE); WITT, Reiner, D-68789 St. Leon-Rot (DE)
(74) Vertreter: Kinzebach, Werner, Dr.
(86) Internationale Anmeldenummer: PCT/EP1999/009462
(87) Internationale Veröffentlichungsnummer: WO 2000/033819

(56) Entgegenhaltungen:
- EP-A- 0 359 195
- EP-A- 0 482 576
- EP-A- 0 559 463
- DE-A- 4 400 295
- GB-A- 2 241 889

## Beschreibung

Die vorliegende Erfindung betrifft ein Verfahren zur Herstellung von Agglomeraten mit Kern-Schale-Struktur und die danach erhältlichen Agglomerate.

Unter Agglomerierung soll vorliegend ein Vorgang verstanden werden, bei dem feinkörnige Stoffe durch Benetzen mit einer Flüssigkeit unter gleichzeitiger mechanischer Bewegung zu Konglomeraten, d.h. Krümeln oder Granalien, zusammengeballt werden. Bei den erhaltenen Agglomeraten handelt es sich um Teilchenverbände, in denen die ursprünglichen Teilchen nicht vollständig miteinander verschmolzen sind, wobei z.B. durch mikroskopische Untersuchung die äußeren Umrisslinien der einzelnen Teilchen noch zum Teil erkennbar sind. Ein agglomeriertes Gut weist im Gegensatz zu dem feinteiligen Ausgangsprodukt zahlreiche Vorteile auf. So lassen sich bestimmte Eigenschaften, wie Festigkeit, Größe, Form und Porosität, definiert einstellen, was sich bei der Lagerung, Förderung und Dosierung vorteilhaft bemerkbar macht. Entmischungserscheinungen werden verhindert, die Schütteigenschaften werden verbessert und die Staubentwicklung beim Verpacken und Umfüllen wird vermindert. Diese Qualitätssteigerung macht den zusätzlichen Bearbeitungsschritt der Agglomeration bei der Herstellung zahlreicher Produkte, wie z.B. Arzneimitteln, rentabel.

Bei der sogenannten Aufbauagglomeration lagern sich die Teilchen beim Benetzen mit Flüssigkeit unter der Wirkung von Grenzflächenkräften bei mechanischer Bewegung des Gutes selbständig aneinander.

Eine Sonderform der Aufbauagglomeration ist die umbenetzungsagglomeration. Grundlage ist hierbei ein 3-Phasen-Gemisch, das aus dem zu agglomerierenden Feingut, einer Suspensionsflüssigkeit und einer Bindeflüssigkeit besteht, die nicht miteinander mischbar sein dürfen. Die Bindeflüssigkeit muß die Eigenschaft besitzen, das suspendierte Gut besser zu benetzen als die Suspensionsflüssigkeit. Das zu agglomerierende Feingut liegt in suspendierter Form in der Suspensionsflüssigkeit vor. Dann wird die Bindeflüssigkeit eingebracht, die mit den Feingutteilchen in Wechselwirkung tritt und zu einer Kornvergrößerung führt. Das gebildete Granulat wird von der wiederverwendbaren Suspensionsflüssigkeit beispielsweise mittels einer Filtriervorrichtung abgetrennt.

Viele Arzneimittel können in fester Form als Agglomerate verabreicht werden. Diese lösen sich im Körper über einen bestimmten Zeitraum hinweg auf. Geschwindigkeitsbestimmend für die Auflösung ist dabei die für einen Angriff durch die Körperflüssigkeit zur Verfügung stehende Angriffsfläche des Arzneimittels, d.h. die Oberfläche des Arzneimittels. Nimmt die Oberfläche durch den Auflösungsprozess ab, so wird pro Zeiteinheit auch eine geringere Menge Wirkstoff freigesetzt. In den meisten Anwendungsfällen ist jedoch eine gleichbleibende Wirkstoff-Freisetzungsrate gewünscht. In einem Aspekt liegt der vorliegenden Erfindung daher die Aufgabe zugrunde, ein Verfahren zur Herstellung von Agglomeraten anzugeben, die eine im Wesentlichen gleichförmige Wirkstoff-Freisetzung über die gesamte Auflösungsdauer aufweisen.

Oft ist es wünschenswert, feste Arzneiformen mit mehr als einem Wirkstoff herzustellen, wobei die Wirkstoffe zu unterschiedlichen Zeitpunkten freigesetzt werden.

In einem anderen Aspekt liegt der Erfindung daher die Aufgabe zugrunde, ein Verfahren zur Herstellung von Agglomeraten anzugeben, die unterschiedliche Wirkstoffe enthalten, die nacheinander freigesetzt werden.

Die DE-OS 44 00 295 beschreibt ein sphärisches Granulum, das dadurch hergestellt wird, dass Lactoseteilchen in eine Granulier- und Beschichtungsvorrichtung eingebracht werden, die mit einer horizontalen Drehscheibe ausgestattet ist, welche eine glatte Oberfläche für die Kontaktierung mit den Granula hat, und dass Lactoselösung während einer Ausführung einer Drehbewegung der Drehscheibe versprüht wird. Das Granulum ist als Träger für Arzneimittel und Nahrungsmittel geeignet.

Die vorstehenden Aufgaben werden erfindungsgemäß durch ein Verfahren zur Herstellung von Agglomeraten mit Kern-Schale-Struktur gelöst, bei dem man i) Primäragglomerate bereitstellt, die einen ersten teilchenförmigen Feststoff enthalten; ii) einen zweiten teilchenförmigen Feststoff in Gegenwart der Primäragglomerate unter Zusatz einer Bindeflüssigkeit zu Agglomeraten der zweiten Stufe agglomeriert und, gegebenenfalls, iii) einen n-ten (wobei n für eine positive ganze Zahl ≧ 3 steht) Feststoff in Gegenwart der Agglomerate der (n-1)-ten Stufe unter Zusatz einer Bindeflüssigkeit zu Agglomeraten der n-ten Stufe agglomeriert. Der Schritt iii) ist fakultativ. Er kann gewünschtenfalls einfach oder mehrfach wiederholt werden. Der Wert von n beginnt bei 3 und erhöht sich bei jeder Wiederholung um 1.

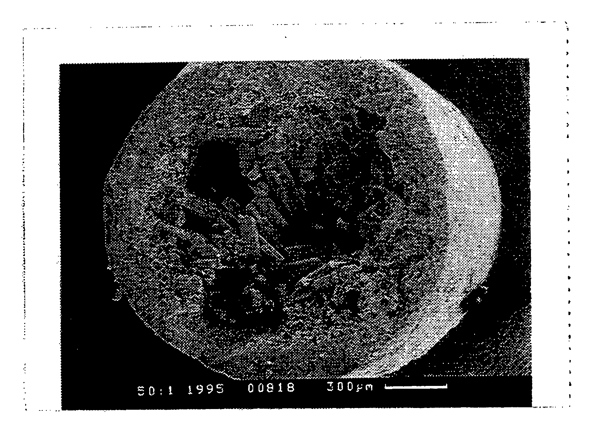

Fig. 1 zeigt eine rasterelektronenmikroskopische Aufnahme eines aufgebrochenen erfindungsgemäß hergestellten Agglomerats.

Beim erfindungsgemäßen Verfahren wird ein teilchenförmiger Feststoff in Gegenwart von Primäragglomeraten agglomeriert. Die Primäragglomerate dienen als Agglomeratkeime. Es wurde überraschenderweise festgestellt, dass es unter diesen Bedingungen möglich ist, dass der teilchenförmige Feststoff sich nicht unter spontaner Keimneubildung an sich selbst, sondern in Form einer Schale um die vorgebildeten Agglomeratkeime anlagert. Die auf diese Weise erhaltenen Agglomerate können wiederum als Agglomeratkeime für die Agglomeration weiterer gleicher oder verschiedener Feststoffe dienen.

Die erfindungsgemäß erhaltenen Agglomerate enthalten einen Kern und wenigstens eine um den Kern angeordnete Schale. Ist mehr als eine Schale angestrebt, so wird der fakultative Schritt iii), gegebenenfalls wiederholt, durchgeführt. Auf diese Weise können Kern-Schale-Agglomerate mit zwei oder mehreren, beispielsweise drei, vier, fünf usw. Schalen um einen Kern erhalten werden. Das Gewichtsverhältnis von Kern zu erster Schale bzw. das Gewichtsverhältnis der innenliegenden Schale zur außenliegenden Schale eines Paars aufeinanderfolgender Schalen liegt vorzugsweise im Bereich von 1:9 bis 9:1, insbesondere 1:9 bis 8:2.

Das erfindungsgemäße Verfahren kann als herkömmliche Aufbauagglomeration durchgeführt werden, wobei Agglomeratkeime und teilchenförmiger Feststoff, gegebenenfalls unter Zugabe der nachstehend erörterten Hilfsstoffe, unter Benetzen mit der Bindeflüssigkeit mechanischer Bewegung ausgesetzt werden. Hierzu sind alle gängigen Mischertypen, wie Konusmischer, Horizontal- oder Vertikalmischer oder Trommelmischer, gegebenenfalls mit einem Zerhacker, geeignet.

Alternativ kann das erfindungsgemäße Verfahren in Form einer Umbenetzungsagglomeration durchgeführt werden. Dabei findet der Vorgang des Zusammenballens des teilchenförmigen Feststoffs in der flüssigen Phase statt. Agglomeratkeime, teilchenförmiger Feststoff und gegebenenfalls Hilfsstoffe werden in einer Suspensionsflüssigkeit verteilt. Die Suspensionsflüssigkeit muß so gewählt sein, dass sowohl Agglomeratkeime als auch der teilchenförmige Feststoff darin im Wesentlichen unlöslich sind. In diese Suspension wird die Bindeflüssigkeit eingebracht. Bindeflüssigkeit und Suspensionsflüssigkeit müssen so gewählt sein, dass sie im Wesentlichen nicht miteinander mischbar sind. Das Einbringen der Bindeflüssigkeit kann unmittelbar über eine Düse oder in Form einer Emulsion aus Binde- und Suspensionsflüssigkeit erfolgen.

Die Bindeflüssigkeit muß so ausgewählt sein, dass sie den teilchenförmigen Feststoff besser benetzt, als die Suspensionsflüssigkeit. Unter Einwirkung der Bindeflüssigkeit treten die Feststoffteilchen in Wechselwirkung und agglomerieren in Form einer Schale um die Agglomeratkeime. Zur Durchführung der Umbenetzungsagglomeration ist z.B. ein Rührkessel oder ein kontinuierlich betriebener Zylinderrührer, wie er beispielsweise in der EP-A-0 690 026 beschrieben ist, geeignet. Die Abtrennung der gebildeten Agglomerate von der Suspensionsflüssigkeit kann über eine beliebige hierzu geeignete Vorrichtung, beispielsweise eine Filtriervorrichtung, erfolgen. Die Agglomerate können anschließend getrocknet werden.

Die Art der jeweiligen teilchenförmigen Feststoffe ist für die vorliegende Erfindung nicht kritisch und unterliegt keinen wesentlichen Einschränkungen. Soweit vorliegend von einem "teilchenförmigen Feststoff" die Rede ist, kann es sich auch um ein Gemisch von Feststoffen handeln. "Teilchenförmig" bedeutet, dass der Feststoff in Form diskreter Partikel vorliegt, die vor der Agglomeration im Wesentlichen keinen Wechselwirkungen untereinander unterliegen. Oft handelt es sich um harte, spröde, nichtklebrige, nicht ohne weiteres verformbare Substanzen. Als Beispiele lassen sich Salze, wie Kaliumchlorid, Ammoniumsulfat, Calciumphosphat, Diammoniumphosphat, Kaliumphosphat, Calciumcarbonat; molekulare organische Verbindungen, wie Harnstoff, Theophyllin, Verapamil; dreidimensional vernetzte Verbindungen, wie Zeolithe; polymere Verbindungen, wie Polyethylenglycole mit einem Molekulargewicht von z.B. 6000 bis 9000, Polymere oder Copolymere von ethylenisch ungesättigten Mono- und Dicarbonsäuren, wie (Meth)acrylsäure oder Maleinsäure, und modifizierte, z.B. ganz oder teilweise neutralisierte , Varianten davon; usw. nennen.

Die Teilchengröße kann in weiten Bereichen variieren. Als allgemeiner Rahmen läßt sich ein Bereich von 1 bis 1000 µm, vorzugsweise 1 bis 500 µm, anführen. Geeignete Teilchengrößen lassen sich durch Kristallisation, mechanische Zerkleinerung kompakter Formen, z.B. durch Mahlung, Zerhacken, Zerstoßen und Sublimation, erhalten.

In bevorzugten Ausführungsformen gelangt im Kern und/oder wenigstens einer Schale ein pharmazeutischer Wirkstoff, gegebenenfalls im Gemisch mit Hilfsstoffen, als teilchenförmiger Feststoff zum Einsatz. Unter pharmazeutischem Wirkstoff im Sinne der Erfindung sind alle Substanzen mit einer angestrebten Wirkung auf den menschlichen oder tierischen Organismus oder Pflanzen zu verstehen. Es können auch Wirkstoff-Kombinationen eingesetzt werden.

Bevorzugte Beispiele von Wirkstoffen, die im Rahmen der Erfindung eingesetzt werden können, sind unter anderem Verapamil, Theophyllin, Ibuprofen, Ketoprofen, Flurbiprofen, Acetylsalicylsäure, Paracetamol, Nifedipin oder Captopril.

Die teilchenförmigen Feststoffe, insbesondere Wirkstoffe, können zum Aufbau des Kerns und/oder einer Schale auch im Gemisch mit Hilfsstoffen vorliegen. Geeignete Hilfsstoffe sind die nachstehend erörterten Bindemittel. Weitere verwendbare Hilfsstoffe sind Streckmittel bzw. Füllstoffe, wie Silikate oder Kieselerde, Magnesiumoxid, Titandioxid, Methylcellulose, Natrium-Carboxymethylcellulose, Talkum, Saccharose, Lactose, Getreide- oder Maisstärke, Kartoffelmehl, Polyvinylalkohol, Aerosil, usw. Ferner können Farbstoffe, Netz-, Konservierungs- und Sprengmittel zugesetzt werden.

Die im Kern und in der ersten Schale beziehungsweise in aufeinanderfolgenden Schalen enthaltenen teilchenförmigen Feststoffe unterscheiden sich vorzugsweise in mindestens einer chemischen und/oder physikalischen Eigenschaft.

So kann es sich bei diesen Feststoffen um unterschiedliche chemische Spezies handeln. Es kann sich auch um Gemische unterschiedlicher Zusammensetzung bzw. einen Reinstoff und ein Gemisch handeln. Ein typisches Anwendungsbeispiel hierfür ist z.B. das Aufbringen eines Gemisches eines Farbstoffes mit einem oder mehreren Wirk- und/oder Hilfsstoffen auf einen keinen Farbstoff enthaltenden Kern.

In anderen Ausführungsformen unterscheiden sich die Feststoffe im Kern und in der ersten Schale bzw. in zwei aufeinanderfolgenden Schalen in mindestens einer physikalischen Eigenschaft, insbesondere in der Teilchengröße. Diese Ausführungsformen sind dadurch gekennzeichnet, dass der erste und zweite teilchenförmige Feststoff und/oder für mindestens einen Wert von n der (n-1)-te und der n-te Feststoff unterschiedliche Teilchengrößen aufweisen.

So können beispielsweise stofflich identische Feststoffe mit mindestens einer unterschiedlichen physikalischen Eigenschaft, z.B. unterschiedlicher Teilchengröße, eingesetzt werden. Dies ist z.B. in Fällen interessant, wo eine gleichförmige Freisetzungscharakteristik von Wirkstoffen beim Auflösen der erfindungsgemäß hergestellten Agglomerate angestrebt ist. So ist es beispielsweise möglich, einen porösen Kern aus Feststoffen mit größerer Teilchengröße und eine höherverdichtete Hülle aus Feststoffen einer geringeren Teilchengröße zu vereinen. Der poröse Kern würde die abnehmende Oberfläche bei der Auflösung des Agglomerats kompensieren und gewährleisten, dass die Freisetzungsrate nicht signifikant abnimmt. Im Allgemeinen ist es bevorzugt, dass der Feststoff in der ersten Schale eine kleinere Teilchengröße als der Feststoff im Kern bzw. der Feststoff in einer außenliegenden Schale eine kleinere Teilchengröße als der Feststoff in der darunterliegenden Schale aufweist.

Vorzugsweise weist der zweite teilchenförmige Feststoff daher eine kleinere Teilchengröße als der erste teilchenförmige Feststoff und/oder für mindestens einen Wert von n, insbesondere für alle Werte, die n einnehmen kann, der n-te teilchenförmige Feststoff eine kleinere Teilchengröße als der (n-1)-te teilchenförmige Feststoff auf.

Vorzugsweise weist der erste teilchenförmige Feststoff in der Richtung der längsten Ausdehnung eine Teilchengröße von 50-800 µm, insbesondere 100-500 µm, auf. Der zweite teilchenförmige Feststoff weist vorzugsweise eine Teilchengröße von weniger als 500 µm, insbesondere weniger als 50 µm, auf. Im Allgemeinen weist der zweite teilchenförmige Feststoff eine Teilchengröße von mindestens 1 µm auf.

Im Einzelfall können erster und zweiter teilchenförmiger Feststoff bzw. die teilchenförmigen Feststoffe aufeinanderfolgender Schalen identisch sein. So kann es vorteilhaft sein, anstelle einer Schale mit großer Schichtdicke mehrere Schalen mit geringerer Schichtdicke aufzubringen. Weitere Vorteile können bisweilen erhalten werden, wenn die Agglomeratkeime vor der weiteren Agglomeration einer Größensortierung unterworfen werden, wobei nur Keime einer bestimmten Größe bzw. eines bestimmten Größenbereichs weiterverwendet werden. Auf diese Weise können Agglomerate mit einer engeren Größenverteilung erhalten werden, als in einer einstufigen Agglomeration.

Die Agglomeration erfolgt unter Zusatz einer Bindeflüssigkeit. Wird beim erfindungsgemäßen Verfahren mehr als eine Schale um einen Agglomeratkeim herum aufgebaut, so können die in den einzelnen Schritten eingesetzten Bindeflüssigkeiten gleich oder verschieden sein. Die Bindeflüssigkeit kann in Abhängigkeit vom zu agglomerierenden Feststoff aus einem breiten Bereich von Flüssigkeiten ausgewählt werden. Wesentlich ist, dass die Bindeflüssigkeit in der Lage ist, den zu agglomerierenden Feststoff ausreichend zu benetzen. Als Maß für die Benetzungsfähigkeit einer Bindeflüssigkeit läßt sich ein Kontaktwinkel δ, der auch als Rand- oder Benetzungswinkel bezeichnet wird, definieren, den die Flüssigkeit mit der Oberfläche des Feststoffs bildet. Vorzugsweise ist der Kontaktwinkel δ kleiner 90°, insbesondere kleiner 60°.

Die Bindeflüssigkeit kann so gewählt sein, dass der zu agglomerierende Feststoff in ihr löslich ist. Es versteht sich, dass in diesem Fall die Bindeflüssigkeit lediglich in einer Menge verwendet werden kann, die nicht zur vollständigen Lösung des zu agglomerierenden Feststoffs ausreicht. Im Allgemeinen werden weniger als 20 Gew.-%, vorzugsweise 1 bis 15 Gew.-%, bezogen auf das Gewicht des zu agglomerierenden Feststoffs, an lösender Bindeflüssigkeit eingesetzt. Bei der Agglomeration löst die zugesetzte Bindeflüssigkeit den zu agglomerierenden Feststoff an, wobei nach dem Trocknen der Agglomerate Kristallbrücken zwischen den Teilchen zurückbleiben, die den Zusammenhalt der Teilchen bewirken.

Insbesondere für den Fall, dass der zu agglomerierende Feststoff in der Bindeflüssigkeit nur gering löslich oder unlöslich ist, kann der Bindeflüssigkeit und/oder dem zu agglomerierenden Feststoff eine in der Bindeflüssigkeit lösliche Substanz zugesetzt werden, die nach dem Trocknen der Agglomerate zu Materialbrücken zwischen den Teilchen führt. Hierzu sind z.B. organische und anorganische Salze, wie Natriumchlorid, Kaliumchlorid, Kaliumnitrat, Natriumnitrat oder Natriumacetat, bei Raumtemperatur feste organische Säuren, wie Ascorbinsäure, Zitronensäure, Adipinsäure; Zucker, z.B. Monosaccharide, wie Glucose, Fructose; Di- oder Oligosaccharide, wie Saccharose oder Lactose; oder Harnstoff geeignet.

In bevorzugten Ausführungsformen wird der Bindeflüssigkeit und/oder dem zu agglomerierenden Feststoff ein polymeres Bindemittel zugesetzt. Beispiele für geeignete polymere Bindemittel sind Polyethylenglycole, Polypropylenglycole und gemischte Polymere davon, Polyvinyllactame, insbesondere Polyvinylpyrrolidon (PVP), Copolymere von vinyllactamen, wie N-Vinylpyrrolidon, N-Vinylpiperidon und N-Vinyl-ε-caprolactam, N-Vinylpyrrolidon mit (Meth)acrylsäure, (Meth)acrylsäureestern, Vinylestern, insbesondere Vinylacetat, Copolymerisate von Vinylacetat und Crotonsäure, teilverseiftes Polyvinylacetat, Polyvinylalkohol, Polyhydroxyalkylacrylate, Polyhydroxyalkyl(meth)acrylate, Polyacrylate und Poly(meth)acrylate, Copolymerisate von Methyl(meth)acrylat und Acrylsäure, Celluloseester, Celluloseether, insbesondere Methylcellulose und Ethylcellulose, Hydroxyalkylcellulosen, insbesondere Hydroxypropylcellulose, Hydroxyalkyl-alkylcellulosen, insbesondere Hydroxypropyl-ethylcellulose, Cellulosephthalate, insbesondere Celluloseacetatphthalat und Hydroxypropylmethylcellulosephthalat, und Mannane, insbesondere Galactomannane. Brauchbar sind auch biologisch abbaubare Polymere, wie Polyhydroxyalkanoate, z.B. Polyhydroxybuttersäure, Polymilchsäure, Polyaminosäuren, z.B. Polylysin, Polyasparagin, Polydioxane und Polypeptide. Die eingesetzten polymeren Bindemittel können je nach Art des zu agglomerierenden Feststoffs hydrophil oder hydrophob sein. Die Bindemittel können in der jeweiligen Bindeflüssigkeit löslich oder dispergiert bzw. dispergierbar sein.

Beim erfindungsgemäßen Verfahren werden Primäragglomerate vorgelegt, die einen ersten teilchenförmigen Feststoff enthalten. Diese können nach einem beliebigen Agglomerationsverfahren hergestellt werden. Es ist bevorzugt, dass die Primäragglomerate durch Agglomeration des ersten teilchenförmigen Feststoffs mittels einer Aufbauagglomeration oder Umbenetzungsagglomeration unter Verwendung einer Bindeflüssigkeit hergestellt werden. Hierzu kann auf die vorstehenden Ausführungen verwiesen werden, wobei jedoch die Agglomeration des ersten teilchenförmigen Feststoffs in Abwesenheit von Agglomeratkeimen unter spontaner Keimbildung erfolgt. Es ist besonders vorteilhaft, die Agglomeration des ersten teilchenförmigen Feststoffs zu Primäragglomeraten in der gleichen Vorrichtung vorzunehmen, in der anschließend die Anlagerung des zweiten und gegebenenfalls weiterer teilchenförmiger Feststoffe erfolgt. So kann der erste teilchenförmige Feststoff vorgelegt und unter Zugabe einer Bindeflüssigkeit zu Primäragglomeraten agglomeriert werden. Unter Zugabe weiterer Bindeflüssigkeit bzw. einer anderen Bindeflüssigkeit und des zweiten teilchenförmigen Feststoffs erfolgt dessen Agglomeration unter Ausbildung einer Schale um die als Agglomeratkeime dienenden Primäragglomerate. Sind weitere Schalen angestrebt, so wird die vorstehende Vorgehensweise einfach oder mehrfach wiederholt.

Die Primäragglomerate weisen üblicherweise eine Größe von 50 bis 1500, vorzugsweise von 100 bis 1000 µm auf. Insbesondere für den Fall, dass eine enge Agglomeratgrößenverteilung angestrebt ist, können die Primäragglomerate einer Sortierung nach der Größe unterworfen werden. Es werden nur Primäragglomerate einer bestimmten Größe bzw. eines bestimmten Größenbereichs weiterverwendet. Bei Anlagerung weiterer Schalen können die Agglomerate der vorhergehenden Stufe ebenfalls einer Größenfraktionierung unterworfen werden.

Das erfindungsgemäße Verfahren besitzt vielfältige Anwendungsmöglichkeiten. Zur Anwendung im pharmazeutischen Bereich können z.B. Agglomerate mit einem vom Mittelpunkt radial nach außen veränderlichen Dichtegradienten hergestellt werden, bei denen die freigesetzte Menge an Wirkstoff nicht nur über den Durchmesser, sondern auch über eine Durchmesser-abhängige Porosität beeinflußt werden kann. Ferner können nach dem erfindungsgemäßen Verfahren feste Arzneiformen hergestellt werden, in denen zwei unterschiedliche Wirkstoffe kombiniert sind, die nacheinander freigesetzt werden. Zum Beispiel kann der Wirkstoff in einer äußeren Schale im Magen, der Wirkstoff in einer inneren Schale, bzw. im Kern, im Darm gelöst werden. Nach dem erfindungsgemäßen Verfahren können auch inkompatible Wirkstoffe zu einer einheitlichen Dosierungsform verarbeitet werden.

Im Bereich der Waschhilfsmittel können z.B. ein Inkrustungsinhibitor und ein Bleichmittel schalenartig auf einen Kern aus einem Tensid aufgebracht werden. Die Erfindung wird durch die folgenden Beispiele näher veranschaulicht.

### Beispiel 1

Dieses Beispiel veranschaulicht die Herstellung eines Kern-Schale-Agglomerats mit kristallinem Theophyllin (mittlere Teilchengröße 100 µm) im Kern und pulverförmigem Theophyllin (mittlere Teilchengröße 10 µm) in der Schale. Die Agglomeration erfolgt in Form einer Umbenetzungsagglomeration mit Cyclohexan als Suspensionsflüssigkeit und Wasser als Bindeflüssigkeit.

Die Agglomeration erfolgte in einem doppelwandigen Glasbehälter mit einem Fassungsvermögen von 2 l unter Umgebungsbedingungen. Als Rührorgan diente ein nach oben fördernder Vierblattrührer, dessen Flügel mit einem Einstellwinkel von 45° um 90° versetzt angebracht waren. Als Stromstörer wurden 3 Blätter mit einer Breite von 15 mm in den Behälter eingeführt.

Es wurden 700 ml Cyclohexan und 140 g kristallines Theophyllin in den Behälter eingefüllt. Das kristalline Theophyllin wurde im Cyclohexan unter Rühren mit 700 U/min gleichmäßig verteilt. Durch eine Düse mit einem Durchmesser von 1,4 mm, die 6 cm unterhalb der Suspensionsoberfläche angebracht war, wurden über 30 min verteilt 73 ml vollentsalztes Wasser unter einem Druck von 2 bar eingebracht. Die so hergestellten Keime wurden in der Suspension belassen.

Unter ständigem Rühren mit 700 U/min wurde die Suspension mit 700 ml Cyclohexan verdünnt. Dann wurden 280 g pulverförmiges Theophyllin über einen Trichter in den Behälter eingebracht. Anschließend wurden unter den gleichen Bedingungen wie zuvor 109,2 ml vollentsalztes Wasser über 45 min dazugegeben.

Anschließend wurde der Rührbehälter demontiert und sein Inhalt über eine Nutsche abfiltriert. Die Agglomerate wurden an der Luft getrocknet. Es wurden Kern-Schale-Agglomerate mit einem porösen Kern aus kristallinem Theophyllin und einer verdichteten Hülle aus pulverförmigem Theophyllin erhalten. Figur 1 zeigt eine rasterelektronenmikroskopische Aufnahme eines aufgebrochenen Agglomerats.

### Beispiel 2

Es wurden Kern-Schale-Agglomerate mit einem Kern aus Calciumcarbonat (Teilchengröße etwa 4 µm) und einer Schale aus einem Gemisch aus Calciumcarbonat (Teilchengröße etwa 4 µm) und Riboflavin C hergestellt. Sowohl Kern als auch Schale enthielten als Bindemittel Polyvinylpyrrolidon (K-Wert 30).

1000 g Calciumcarbonat sowie 50 g Polyvinylpyrrolidon wurden im Mischertopf eines Eirich-Mischers vorgelegt und bei maximalem Energieeintrag 2 min vorgemischt. Unter maximalem Energieeintrag wurden über 40 s verteilt 160 g Wasser mit einer Spritzflasche hinzugefügt. Anschließend wurde weitere 10 s ohne weitere Flüssigkeitszufuhr weitergemischt.

4000 g Calciumcarbonat, 300 g Riboflavin C sowie 215 g Polyvinylpyrrolidon wurden 5 min lang vorgemischt. 1050 g dieses Gemisches wurden zu den wie vorstehend hergestellten Agglomeratkeimen gegeben. Man mischte 48 s mit einem Sternwirbler bei einer Wirblerdrehzahl von 1500 U/min und Tellerdrehzahl von 84 U/min mit gegensinniger Fahrweise zwischen Topf und Wirbler. Innerhalb von 25 s wurden 100 g Wasser mittels einer Spritzflasche dazugegeben, anschließend wurde 74 s weitergemischt. Es wurden Kern-Schale-Agglomerate mit einem Kern aus ungefärbtem Calciumcarbonat und einer Schale aus gelb eingefärbtem Calciumcarbonat erhalten.

## Patentansprüche

1. Verfahren zur Herstellung von Agglomeraten mit Kern-Schale-Struktur, bei dem man
i) Primäragglomerate bereitstellt, die einen ersten teilchenförmigen Feststoff enthalten;
ii) einen zweiten teilchenförmigen Feststoff in Gegenwart der Primäragglomerate unter Zusatz einer Bindeflüssigkeit zu Agglomeraten der zweiten Stufe agglomeriert und, gegebenenfalls,
iii) einen n-ten (n≧3) Feststoff in Gegenwart der Agglomerate der (n-1)-ten Stufe unter Zusatz einer Bindeflüssigkeit zu Agglomeraten der n-ten Stufe agglomeriert, wobei die Agglomeration in Schritt ii) und/oder iii) in einer Suspensionsflüssigkeit erfolgt, in der sowohl der erste teilchenförmige Feststoff als auch der zweite teilchenförmige Feststoff bzw. der (n-1)-te teilchenförmige Feststoff und der n-te teilchenförmige Feststoff im Wesentlichen unlöslich sind, und die mit der Bindeflüssigkeit im Wesentlichen nicht mischbar ist.

2. Verfahren nach Anspruch 1, **dadurch gekennzeichnet, dass** der erste und zweite teilchenförmige Feststoff und/oder für mindestens einen Wert von n der (n-1)-te und der n-te teilchenförmige Feststoff unterschiedliche Teilchengrößen aufweisen.

3. Verfahren nach Anspruch 2, **dadurch gekennzeichnet, dass** der zweite teilchenförmige Feststoff eine kleinere Teilchengröße als der erste teilchenförmige Feststoff und/oder für mindestens einen Wert von n der n-te teilchenförmige Feststoff eine kleinere Teilchengröße als der (n-1)-te teilchenförmige Feststoff aufweist.

4. Verfahren nach Anspruch 2 oder 3, **dadurch gekennzeichnet, dass** der erste und zweite und/oder für mindestens einen Wert von n der (n-1)-te und n-te teilchenförmige Feststoff stofflich identisch sind.

5. Verfahren nach einem der vorhergehenden Ansprüche, **dadurch gekennzeichnet, dass** der erste teilchenförmige Feststoff eine Teilchengröße von 50-800 µm aufweist.

6. Verfahren nach einem der vorhergehenden Ansprüche, **dadurch gekennzeichnet, dass** der zweite teilchenförmige Feststoff eine Teilchengröße von weniger als 500 µm aufweist.

7. Verfahren nach einem der vorhergehenden Ansprüche, **dadurch gekennzeichnet, dass** die Agglomeration in Schritt ii) und/oder iii) in Gegenwart einer brückenbildenden Substanz und/oder eines Bindemittels erfolgt.

## Claims

1. A process for producing agglomerates with a core/shell structure, in which
i) initial agglomerates containing a first particulate solid are prepared;
ii) a second particulate solid is agglomerated in the presence of the initial agglomerates with the addition of a binder liquid to give second stage agglomerates and, where appropriate,
iii) an nth (n ≥ 3) solid is agglomerated in the presence of the (n-1)th stage agglomerates with the addition of a binder liquid to give nth stage agglomerates, the agglomeration in step ii) and/or iii) taking place in a suspending liquid in which both the first particulate solid and the second particulate solid or the (n-1)th particulate solid and the nth particulate solid are essentially insoluble, and which is essentially immiscible with the binder liquid.

2. A process as claimed in claim 1, wherein the first and second particulate solid and/or for at least one value of n the (n-1)th and the nth particulate solid have different particle sizes.

3. A process as claimed in claim 2, wherein the second particulate solid has a smaller particle size than the first particulate solid and/or for at least one value of n the nth particulate solid has a smaller particle size than the (n-1)th particulate solid.

4. A process as claimed in claim 2 or 3, wherein the first and second and/or for at least one value of n the (n-1)th and nth particulate solid comprise identical substances.

5. A process as claimed in any of the preceding claims, wherein the first particulate solid has a particle size of 50-800 µm.

6. A process as claimed in any of the preceding claims, wherein the second particulate solid has a particle size of less than 500 µm.

7. A process as claimed in any of the preceding claims, wherein the agglomeration in step ii) and/or iii) takes place in the presence of a bridge-forming substance and/or of a binder.

## Revendications

1. Procédé pour la préparation d'agglomérats à structure en noyau-enveloppe dans lequel
i) on prépare des agglomérats primaires qui contiennent une première substance solide à l'état de particules ;
ii) avec une deuxième substance solide à l'état de particules, en présence des agglomérats primaires et avec adjonction d'un liquide liant, on forme des agglomérats du deuxième stade et, le cas échéant,
iii) avec une n-ième (n supérieur ou égal à 3) substance solide, en présence des agglomérats du (n-1)ième stade et avec adjonction d'un liquide liant, on forme les agglomérats du n-ième stade, les formations d'agglomérats des stades ii) et/ou iii) étant réalisées dans un liquide de suspension dans lequel à la fois la première substance solide à l'état de particules et la deuxième substance solide à l'état de particules ou la (n-1)ième substance solide à l'état de particules et la n-ième substance solide à l'état de particules sont essentiellement insolubles, et qui est essentiellement non miscible avec le liquide liant.

2. Procédé selon la revendication 1, **caractérisé en ce que** la première et la deuxième substance solide à l'état de particules et/ou, pour au moins une valeur de n, la (n-1)-ième et la n-ième substance solide à l'état de particules, ont des dimensions de particules différentes.

3. Procédé selon la revendication 2, **caractérisé en ce que** la deuxième substance solide à l'état de particules a une dimension de particule plus faible que la première substance solide à l'état de particules et/ou, pour au moins une valeur de n, la n-ième substance solide à l'état de particules a une dimension de particule inférieure à celle de la (n-1)-ième substance solide à l'état de particules.

4. Procédé selon la revendication 2 ou 3, **caractérisé en ce que** la première et la deuxième et/ou, pour au moins une valeur de n, la (n-1)-ième et la n-ième substance solide à l'état de particules, sont de nature identique.

5. Procédé selon l'une des revendications qui précèdent, **caractérisé en ce que** la première substance solide à l'état de particules a une dimension de particule de 50 à 800 µm.

6. Procédé selon l'une des revendications qui précèdent, **caractérisé en ce que** la deuxième substance solide à l'état de particules a une dimension de particule inférieure à 500 µm.

7. Procédé selon l'une des revendications qui précèdent, **caractérisé en ce que** les formations d'agglomérats des stades ii) et/ou iii) sont réalisées en présence d'une substance formant des ponts et/ou d'un liant.
